**Europäisches Patentamt**

**European Patent Office**

**Office européen des brevets**

(19)

(11) Veröffentlichungsnummer: **0 004 015**
**B1**

(12)

# EUROPÄISCHE PATENTSCHRIFT

(45) Veröffentlichungstag der Patentschrift:
**07.01.81**

(21) Anmeldenummer: **79100567.1**

(22) Anmeldetag: **26.02.79**

(51) Int. Cl.³: **C 07 C 89/02,** C 07 C 89/04,
C 07 C 91/04

(54) **Verfahren zur Herstellung von ungefärbten technischen Äthanolaminen.**

(30) Priorität: **09.03.78 DE 2810135**

(43) Veröffentlichungstag der Anmeldung:
**19.09.79 Patentblatt 79/19**

(45) Bekanntmachung des Hinweises auf die Patenterteilung:
**07.01.81 Patentblatt 81/1**

(84) Benannte Vertragsstaaten:
**BE DE FR GB NL**

(56) Entgegenhaltungen:
**FR-A-2 138 902**
**US-A-3 131 132**
**US-A-3 151 166**

(73) Patentinhaber: **BASF Aktiengesellschaft,**
**Carl-Bosch-Strasse 38, D-6700 Ludwigshafen (DE)**

(72) Erfinder: **Bosche, Horst Guenther, Dr.,**
**Julius-Leber-Strasse 23, D-6720 Speyer (DE)**
Erfinder: **Hammer, Hans, Waldlichtung 42,**
**D-6800 Mannheim 1 (DE)**
Erfinder: **Jeschek, Gerhard, Dr., Im Zaunruecken 14,**
**D-6718 Gruenstadt 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

ACTORUM AG.

# Verfahren zur Herstellung von ungefärbten technischen Äthanolaminen

Es wurde gefunden, dass man Äthanolamine nach dem üblichen Verfahren, d.h. durch Umsetzung von Ammoniak und Äthylenoxid in hervorragender Farbqualität erhält, wenn man eine wirksame Menge phosphorige oder unterphosphorige Säure bzw. deren Derivate vor oder während oder nach der Umsetzung, jedenfalls aber vor der Isolierung der einzelnen Äthanolamine zusetzt, so dass diese Mittel während der Destillation, mit deren Hilfe die Isolierung vorgenommen wird, anwesend sind. Gemeint ist beispielsweise die Zugabe der genannten Phosphorverbindungen zu dem in die Oxäthylierungsreaktion eingesetzten wässrigen Ammoniak oder die Zugabe zu dem ausreagierenden, gegebenenfalls nur von Ammoniak oder auch von Wasser befreiten Äthanolamin-Gemisch. Auch eine verteilte Zugabe auf die einzelnen Prozessstufen ist möglich und mitunter vorteilhaft. Zur Anwendung kommen die Phosphorverbindungen in einer Konzentration von 0,01 bis 2 Gew.%, bezogen auf die Summe der Äthanolamine; die Wirkung tritt auch bei noch grösseren Zusätzen ein, jedoch spielt dann die Frage der Wirtschaftlichkeit eine Rolle.

Phosphorige bzw. unterphosphorige Säure, die im allgemeinen durch die Formel $H_3PO_3$ bzw. $H_3PO_2$ wiedergegeben werden, können in monomerer oder gegebenenfalls polymerer Form, in wasserhaltiger Form (Hydrate), als Anlagerungsverbindungen, Salze und Ester zugegeben werden.

Zwar ist aus der DE-AS 2 435 792 bekannt, dass man bei der Destillation von Diolen, die aus den bei der Cyclohexanoxidation als Nebenprodukte anfallenden Carbonsäuren durch Hydrierung erzeugt werden, Destillate von guter farblicher Qualität erhält, wenn man dem – bereits verfärbten – Destillationsgut phosphorige Säure in katalystischer Menge zusetzt. Versucht man nun, diese Erfahrung auf die Destillation von Äthanolaminen, die durch Anlagerung von Äthylenoxid an Ammoniak nach konventionellen technischen Verfahren hergestellt werden und dabei oftmals in schlechter Farbqualität anfallen, zu übertragen, so gelingt es nicht, bereits mehr oder weniger verfärbte technische Äthanolamine durch eine in Gegenwart von phosphoriger Säure durchgeführte Destillation in befriedigendem Masse zu entfärben.

Es war daher nicht ohne weiteres zu erwarten, dass durch den Zusatz von phosphoriger oder unterphosphoriger Säure zu Äthanolaminen deren Verfärbung verhindert werden kann. Es ist auch nicht anzunehmen, dass die Verfärbung von Diolen und von Äthanolaminen auf den gleichen Ursachen beruht; wie in der DE-PS 2 435 792 ausgeführt ist, tritt die Verfärbung von Diolen auch bei der Lagerung zunächst farbloser Produkte auf. Auch Äthanolamine können sich bei der Lagerung verfärben. Diese Erscheinung beruht wahrscheinlich ebenfalls auf anderen Ursachen wie die Verfärbung von Diolen.

Im Zusammenhang mit der vorliegenden Erfindung ist gefunden worden – was die Erfindung jedoch in keiner Weise begrenzen soll –, dass die Eigenschaften des Wandmaterials der Reaktionsräume eine Rolle spielen. Die Erfindung erlaubt es, auch gewöhnliche Kohlenstoffstähle als Reaktionsmaterial zu verwenden, wie die nachfolgenden Beispiele zeigen.

Beispiel 1:
In je einen Rührautoklaven aus Edelstahl und aus Normalstahl von jeweils 9 l Inhalt werden jeweils 850 destilliertes Wasser und 0,5 g phosphorige Säure (Versuch A) eingefüllt sowie anschliessend 3400 g Ammoniak aus einer Stahlflasche eingepresst. Man heizt den Autoklaveninhalt auf 120 °C auf und pumpt bei dieser Temperatur im Laufe von 30 Minuten 1500 ml Äthylenoxid ein. Im Verlauf von 2 Stunden kühlt man den Autoklaven auf 25 bis 30 °C ab und entspannt anschliessend langsam auf Normaldruck. Der Autoklaveninhalt wird entnommen, von restlichem, überschüssigem Ammoniak befreit, danach das Wasser ebenfalls abgetrieben und das zurückbleibende Äthanolamin-Gemisch unter vermindertem Druck rektifiziert, wobei man das Destillat – abgesehen von Monoäthanolamin, das in einer einzigen Fraktion aufgefangen wird – in 50 ml-Fraktionen aufteilt. Die Farbzahl nach APHA dieser Fraktionen wird gemessen. In jeweils zwei weiteren Versuchen werden die Zusätze von phosphoriger Säure unterlassen («Vergleich») und in einem Versuch B das aus der Umsetzung in Normalstahl ohne Zusatz erhaltene Reaktionsgemisch nach dem Entfernen des Ammoniaks, jedoch vor der Destillation mit 0,5 g phosphoriger Säure versetzt.

Die Tabelle gibt die Farbzahlen der jeweils erhaltenen Fraktionen wieder.

Tabelle 1

| Autoklavenmaterial | Edelstahl | | Normalstahl | | |
| Versuch: | A | Vergleich | A | B | Vergleich |
| --- | --- | --- | --- | --- | --- |
| Monoäthanolamin (MEA) | 5 | 6,5 | 6,5 | 3,5 | 6,5 |
| | 10 | 10,0 | 8,0 | 10,0 | 46,0 |
| | 10 | 10,0 | 7,5 | 5,0 | 26,0 |
| | 10 | 16,0 | 7,5 | 5,0 | 15,0 |
| Diäthanolamin (DEA) | 8 | 16,0 | 7,5 | 8,0 | 15,5 |
| | 4 | 16,0 | 7,5 | 8,0 | 20,0 |
| | 6 | 10,0 | 6,0 | 6,0 | 27,5 |
| | 5 | 10,5 | 6,5 | 7,5 | 33,0 |
| Triäthanolamin (TEA) | 7,5 | 100,0 | 11,0 | 15,0 | 139,5 |
| | 15 | 110,5 | 63,0 | 20,0 | 329,0 |
| | 40 | 72,5 | 265,0 | 53,0 | 362,5 |

Beispiel 2:
In einen 6 l-Rührkolben aus Glas, der mit jeweils 500 g Eisen- bzw. Stahlspänen beschickt ist, werden 4500 g 25%iger, wässriger Ammoniaklösung und wechselnde Mengen (in der Tabelle 2 mit A bezeichnet) phosphorige bzw. unterphosphorige Säure eingefüllt bzw. zum Vergleich weggelassen. Anschliessend wird bei 40°C 1000 g Äthylenoxid gasförmig eingeleitet. Nach Beendigung der Oxäthylierung wird in ausgewählten Versuchen zum ausreagierten Gemisch phosphorige Säure (Buchstabe B in Tabelle 2) hinzugefügt und der überschüssige Ammoniak und das Wasser abdestilliert. Wiederum in einzelnen Versuchen (Buchstabe C in Tabelle 2) werden unterschiedliche Mengen phosphoriger Säure während der Isolierung des Äthanolamin-Gemischs durch Destillation unter vermindertem Druck zugesetzt, wobei man das Destillat – abgesehen von MEA, das in einer einzigen Fraktion aufgefangen wird – in 50 ml-Fraktionen aufteilt. Die Farbzahl nach APHA dieser Fraktionen wird gemessen. Sie ist für insgesamt 8 Versuche, 2 Vergleichsversuche eingeschlossen, in der folgenden Tabelle angegeben.

Tabelle 2

| Metallzusatz | Eisenspäne | Eisenspäne | Eisenspäne | Eisenspäne | Eisenspäne | Eisenspäne | Pulver a.V4A-St. | Pulver a.V4A-St. |
|---|---|---|---|---|---|---|---|---|
| A | 0,5 g $H_3PO_3$ | 0,17 g $H_3PO_3$ | – | 0,5 g $H_3PO_3$ | 1,0 g $H_3PO_2$ | – | 1,0 g $H_3PO_3$ | – |
| B | – | 0,17 g $H_3PO_3$ | 0,25 g $H_3PO_3$ | – | – | – | – | – |
| C | – | 0,17 g $H_3PO_3$ | 0,25 g $H_3PO_3$ | – | – | – (Vgl.) | – | – (Vgl.) |
| MEA | 5 | 4 | 4 | 5 | 4,5 | 6,0 | 6,5 | 9 |
| DEA | 20 | 10 | 10 | 9 | 20 | 22 | 19 | 15 |
| | 15 | 8 | 10 | 9 | 25 | 27,5 | 15 | 20 |
| | 15 | 8 | 10 | 9 | 26,5 | 30 | 6,5 | 25 |
| | 10 | 8 | 7,5 | 6 | 23 | 25 | 4,5 | 20 |
| | 10 | 8 | 6 | 6 | 21 | 26 | 10 | 16 |
| | 7,5 | 8 | 6 | 5 | 23,5 | 25 | 9 | 16 |
| | 11 | 13 | 7,5 | 10 | 36 | 40 | 12 | 43 |
| TEA | 25 | 20 | 8 | 6 | 50 | 55,5 | 30 | 220 |
| | 35 | 30 | 15 | 14 | 40 | 187 | 30 | 216 |
| | 40 | 40 | 20 | 23 | 60 | 322 | 30 | 310 |
| | 30 | 50 | 25 | 23 | 60 | 420 | 30 | |

## Patentanspruch

Verfahren zur Herstellung von Mono-, Di- oder Triäthanolamin durch stufenweise Umsetzung von gegebenenfalls wässrigem Ammoniak mit Äthylenoxid, dadurch gekennzeichnet, dass man die Umsetzung in Gegenwart einer wirksamen Menge von phosphoriger oder unterphosphoriger Säure bzw. deren Derivate vornimmt oder diese Verbindungen dem Gemisch der erhaltenen Äthanolamine vor oder vor und während deren Isolierung durch Destillation zusetzt.

## Revendication

Procédé pour la préparation de mono-, di- ou triéthanolamine par réaction étagée d'ammoniac éventuellement aqueux avec l'oxyde d'éthylène, caractérisé en ce qu'on mène la réaction en présence d'une quantité active d'acide phosphoreux ou hypophosphoreux ou de leurs dérivés, ou bien on ajoute ces composés au mélange des éthanolamines obtenues, avant ou avant et pendant l'isolement de celles-ci par distillation.

## Claim

A process for the preparation of mono-, di- or triethanolamine by reaction in stages of optionally aqueous ammonia with ethylene oxide, characterized in that the reaction is carried out in the presence of an effective amount of phosphorous or hypophosphorous acid or a derivative thereof or any of these compounds is added to the mixture of the resulting ethanolamines before or before and during their isolation by distillation.